# EUROPÄISCHE PATENTSCHRIFT

(11) **EP 2 665 954 B1**
(45) Veröffentlichungstag und Bekanntmachung des Hinweises auf die Patenterteilung: **16.09.2015**
(21) Anmeldenummer: 12707244.5
(22) Anmeldetag: 23.01.2012
(51) Int. Cl.: F16K 31/08, F16K 99/00, H05K 1/02, A61M 13/00, A61M 16/20

(54) **LEITERPLATTE MIT ÜBERDRUCKVENTIL, INSUFFLATOR**
CIRCUIT BOARD HAVING A PRESSURE-RELIEF VALVE, INSUFFLATOR
CARTE DE CIRCUITS IMPRIMÉS À SOUPAPE DE SURPRESSION, INSUFFLATEUR

(30) Priorität: 21.01.2011 DE 102011003007
(43) Veröffentlichungstag der Anmeldung: 27.11.2013
(73) Patentinhaber: MGB Endoskopische Geräte GmbH Berlin, 12489 Berlin (DE)
(72) Erfinder: PAGEL, Lienhard, 18311 Klockenhagen (DE); GASSMANN, Stefan, 18059 Rostock (DE)
(74) Vertreter: Eisenführ Speiser
(86) Internationale Anmeldenummer: PCT/EP2012/050973
(87) Internationale Veröffentlichungsnummer: WO 2012/098257

(56) Entgegenhaltungen:
- DE-A1- 3 621 332
- DE-A1-102009 007 393
- US-A- 4 489 754
- US-B1- 6 299 592

## Beschreibung

Die vorliegende Erfindung betrifft eine Leiterplatte mit wenigstens einem im Inneren der Leiterplatte angeordneten Fluidkanal und einer mehrstückigen Überdruckventilanordnung zum Abbauen eines Überdrucks im Fluidkanal. Außerdem betrifft die vorliegende Erfindung einen Insufflator mit einer solchen Leiterplatte.

Eine Leiterplatte als Träger für elektronische Bauteile, die einen oder mehrere im Inneren der Leiterplatte angeordnete Fluidkanäle umfasst, ist bekannt. Die im Inneren der Leiterplatte angeordneten Fluidkanäle führen beispielsweise ein Fluid, wie eine Flüssigkeit, welche einem Kühlen von auf der Leiterplatte angeordneten elektronischen Bauteilen während des Betriebes dient. Ebenfalls ist der Einsatz einer solchen Leiterplatte bei einem fluidischen Mikrosystem bekannt, bei dem die im Inneren der Leiterplatte angeordneten Fluidkanäle ein Fluid führen, dass zu anderen vordergründigen Zwecken als zur Kühlung von Bauelementen eingesetzt wird. Ein derartiges mikrofluidisches System findet beispielsweise in Gebieten wie der Biotechnologie, der Medizintechnik, der Prozesstechnik, der Sensortechnik aber auch bei Konsumgütern Anwendung.

Eine Leiterplatte mit im Inneren der Leiterplatte angeordneten Fluidkanälen ist beispielsweise aus der Veröffentlichung DE 197 39 722 A1 bekannt. Diese vorbekannte Leiterplatte hat mehrere Schichten, auf denen Leiterzüge so angeordnet sind, dass Zwischenräume entstehen, die als Kanäle für Fluide genutzt werden können. In ausgewählten Schichten sind Aussparungen eingebracht, in die Sensoren eingesetzt werden können, so dass die Sensoren direkten Kontakt zu einem in den Fluidkanälen strömenden Fluid haben können.

Zum Abbauen eines Überdrucks in einem Fluidkanal werden Überdruckventile eingesetzt. Ein derartiges Überdruckventil ist beispielsweise aus der Veröffentlichung: F. Peridigones et al.: "Safty valve in PCB-MEMS technology for limiting pressure in microfluidic applications", 2010 IEEE, International Conference on Industrial Technologie, 14. - 17.März 2010, S. 1558-1561, bekannt. Das aus dieser Veröffentlichung vorbekannte Überdruckventil hat einen brückenartigen Aufbau, bei dem ein biegbarer Spannbügel als zeitweiliger Verschluss über eine Öffnung in der Leiterplatte, die zum Fluidkanal führt, gespannt ist. Erhöht sich der Druck im Fluidkanal und somit auf den Verschluss, so wird der die Öffnung überspannende Teil des Ventils derart in Richtung der Leiterplatte gebogen, so dass die Öffnung blockiert ist und somit der Fluss im betreffenden Fluidkanal unterbrochen wird. Auf diese Weise wird verhindert, dass es zum weiteren Druckaufbau kommt. Insofern wäre das vorbekannte Druckventil eher als ein Druckregler zu bezeichnen, da ein Fluidzufluss über einen Eingang durch Schließen des Eingangs unterbrochen wird und nicht, wie sonst bei einem Ventil üblich, ein Fluidsystem zum Druckabbau geöffnet wird. Nachteilig an dem vorbekannten Überdruckventil sind der aufwendige Aufbau sowie die komplizierte Dimensionierung der beteiligten Bauelemente zur Erzielung des gewünschten Wirkprinzips bei einer konkreten Leiterplatte.

DE 10 2009 007939 A1 offenbart eine Leiterplatte für einen Insufflator, die in ihrem Inneren wenigstens einen Strömungskanal aufweist. Ein Insufflator mit einem Überdruckventil ist der US 6 299 592 B1 entnehmbar. US 4 489 754 A offenbart eine mehrstückige Überdruckventilanordnung zum Abbauen eines Überdrucks in einem Gasbehälter für Kühlzwecke. Die Überdruckventilanordnung weist einen ersten Magneten, einen zweiten Magneten und einen Dichtverschluss auf, wobei der erste Magnet und der zweite Magnet derart angeordnet sind, dass diese den Dichtverschluss durch eine von dem ersten Magneten und von dem zweiten Magneten bewirkte Magnetkraft auf eine Fluidkanalöffnung pressen. DE 36 21 332 A1 offenbart eine Leiterplatte mit wenigstens einem in Inneren der Leiterplatte angeordneten Fluidkanal, und eine auf einer Oberfläche der Leiterplatte angeordnete Fluidkanalöffnung, welche zum Fluidkanal führt, und eine mehrstückige und durch eine elektromagnetische Spule umschaltbare bistabile Ventilanordnung, welche einen ersten Magneten, einen zweiten Magneten und einen Dichtverschluss umfasst, wobei der erste Magnet und der zweite Magnet derart angeordnet sind, dass diese den Dichtverschluss durch eine von dem ersten Magneten und von dem zweiten Magneten bewirkte Magnetkraft auf die Fluidkanalöffnung pressen, wenn die sich die bistabile Ventilanordnung in geschlossenem Zustand befindet.

Es ist eine der vorliegenden Erfindung zu Grunde liegende technische Aufgabe, eine Leiterplatte mit wenigstens einem im Inneren der Leiterplatte angeordneten Fluidkanal vorzuschlagen, die sowohl kostengünstig herstellbar ist als auch eine hohe Betriebssicherheit bietet.

Gemäß einem ersten Aspekt wird diese technische Aufgabe gelöst für eine Leiterplatte mit wenigstens einem in Inneren der Leiterplatte angeordneten Fluidkanal, die folgende Komponenten aufweist:
- eine auf einer Oberfläche der Leiterplatte angeordnete Fluidkanalöffnung, welche zum Fluidkanal führt, und
- eine mehrstückige Überdruckventilanordnung zum Abbauen eines Überdrucks im Fluidkanal, welche einen ersten Magneten, einen zweiten Magneten und einen Dichtverschluss umfasst, wobei
- der erste Magnet und der zweite Magnet derart angeordnet sind, dass diese den Dichtverschluss durch eine von dem ersten Magneten und von dem zweiten Magneten bewirkte Magnetkraft auf die Fluidkanalöffnung pressen, und wobei
- die Überdruckventilanordnung so ausgestaltet ist, dass sie nach einem Überdruck im Fluidkanal selbsttätig wieder in den geschlossenen Zustand übergeht.

Die Erfindung schließt die Erkenntnis ein, dass ein Überdruckventil einer Leiterplatte ein für die Betriebssicherheit wichtiges und mitunter notwendiges Element ist, dass jedoch vorbekannte Lösungen in der Konstruktion aufwendig, in der Dimensionierung kompliziert und im Ergebnis teuer sind. Ein vorbekanntes Überdruckventil auf einer Leiterplatte schmälerte daher prinzipielle Vorteile einer Leiterplatte mit Fluidkanälen, nämlich die kostengünstige und kompakte Verbindung von Fluidik und Elektronik.

Im Unterschied zum vorbekannten Überdruckventil wirkt die erfindungsgemäße Überdruckventilanordnung nicht am Eingang des Fluidkanals, sondern an einem Ausgang, und zwar in dem Sinne, dass die Überdruckventilanordnung bei einem aktuellen Überdruck öffnet und damit verhindert, dass im Fluidkanal ein höherer Druck herrscht, als zum Öffnen der Überdruckventilanordnung erforderlich ist.

Die vorliegende Überdruckventilanordnung mit dem ersten und dem zweiten Magneten sowie mit dem Dichtverschluss stellt eine besonders kostengünstige und kompakte Alternative dar. Die beiden Magneten und der Dichtverschluss können sehr einfach installiert werden und erfüllen ihre technische Funktion, nämlich das Abbauen eines im Fluidkanal herrschenden Überdrucks, in einer gegenüber vorbekannten Lösungen verbesserten Weise. Die Überdruckventilanordnung erfordert insbesondere kein zusätzliches Gehäuse oder ähnliches. Die Überdruckventilanordnung erfordert auch keine besonderen Ventilfassungen, Ventilhalter oder Ventilzentriereinrichtungen. Die Kosten der Überdruckventilanordnung liegen um ein Vielfaches unterhalb der Kosten, die für ein vergleichbares, federbetriebenes Überdruckventil anfallen würden.

Anders als beispielsweise bei einem federbetriebenen Ventil ist die Magnetkraft zwischen dem ersten und dem zweiten Magneten, also die Kraft, mit dem Dichtverschluss auf die Fluidkanalöffnung gepresst wird (im Folgenden auch als Schließkraft bezeichnet), meistens in etwa proportional zum Kehrwert des Quadrats des Abstands zwischen dem ersten und dem zweiten Magneten. Steigt der Druck im Fluidkanal über einen vorbestimmten Höchstdruck, so öffnet sich die Überdruckventilanordnung, wobei sich dazu der Abstand zwischen dem ersten Magneten und dem zweiten Magneten erhöht und somit die Schließkraft abnimmt. Dadurch wird ein weiteres Öffnen bei gegebenenfalls noch steigendem Druck im Fluidkanal begünstigt und somit ein schneller Druckabbau garantiert. So zeigt die Überdruckventilanordnung eine Hysterese-Charakteristik, da sie bei einem Druck schließt, der unterhalb des vorbestimmten Höchstdrucks liegt, bei dem sie öffnet. Daher schließt die vorliegende Überdruckventilanordnung der Leiterplatte sicherer als vorbekannte Ventile, da die Schließkraft mit abnehmendem Abstand zunimmt. Im Gegensatz dazu nimmt beispielsweise bei einem Ventil, das mit einer Feder betrieben ist, die Schließkraft im Falle eines durch einen Überdrucks hervorgerufenen Öffnens des Ventils nicht etwa ab, sondern zu.

Der einfache Aufbau der Überdruckventilanordnung, der im Wesentlichen nur drei Komponenten umfasst, nämlich den ersten Magneten und den zweiten Magneten sowie den Dichtverschluss, führt dazu, dass die Überdruckventilanordnung leicht in die Leiterplatte zu integrieren ist. Ebenfalls ist es möglich, die vorstehend genannten Komponenten der Überdruckventilanordnung bei einer Bestückung der Leiterplatte mit einem Bestückungsautomaten zu installieren, so dass auch hinsichtlich der Installation der Überdruckventilanordnung kein besonderer Aufwand anfällt. Die Vorteile der Leiterplatte mit einem integrierten Fluidkanal, nämlich die kostengünstige und kompakte Verbindung von Fluidik und Elektronik, wird durch das zusätzliche Vorsehen der Überdruckventilanordnung nicht etwa geschmälert, sondern bestätigt.

Ein weiterer Vorteil der erfindungsgemäßen Leiterplatte besteht in einem breiten Wertebereich, in welchem die Magnetkraft zwischen dem ersten Magneten und dem zweiten Magneten einstellbar ist. Beispielsweise kann die Magnetkraft, die im Rahmen der Beschreibung der vorliegenden Erfindung auch als Schließkraft bezeichnet wird, durch die Wahl eines bestimmten Magnetmaterials eingestellt werden oder durch die Form und Dimensionierung des ersten und/oder des zweiten Magneten.

Der erste Magnet kann beispielsweise ein Permanentmagnet sein. Der zweite Magnet kann beispielsweise ebenfalls ein Permanentmagnet sein oder aus ferromagnetischem Material bestehen und/oder ein Weicheisen sein. Die Begriffe "erster Magnet" und "zweiter Magnet" sind im Rahmen der Beschreibung der vorliegenden Erfindung technisch funktional zu verstehen, derart, dass zwischen Ihnen eine Magnetkraft wirkt. Das heißt beispielsweise, dass es ausreichend ist, dass nur der erste oder der zweite Magnet tatsächlich aus magnetischem Material besteht, der andere Magnet aber beispielsweise metallisch sein kann, also z.B. aus ferromagnetischem Material, wie ein Weicheisen.

Ein weiterer Vorteil der Leiterplatte ist, dass die Überdruckventilanordnung nach einem Öffnen auf Grund eines Überdrucks im Fluidkanal schnell wieder geschlossen werden kann. Ein aufwendiges Wiedereinsetzen in Ventilfassungen oder ein Einschrauben oder ähnliche Re-Installationsvorgänge sind nicht notwendig. Hinzu kommt, dass der erste Magnet und der zweite Magnet bevorzugt derart angeordnet sind, dass die Magnetkraft den Dichtverschluss zentrierend auf die Fluidkanalöffnung presst und insofern ein aufwendiges Positionieren ebenfalls entfällt.

Nachfolgend werden einige Ausführungsformen der erfindungsgemäßen Leiterplatte des ersten Aspektes der Erfindung beschrieben. Zusätzliche Merkmale dieser Ausführungsformen können zur Bildung weiterer Ausführungsvarianten miteinander kombiniert werden, sofern sie nicht ausdrücklich als alternativ zueinander bezeichnet sind.

In einer bevorzugten Ausführungsform sind der erste Magnet auf dem beweglichen Dichtverschluss und der zweite Magnet auf einer der Fluidkanalöffnung abgewandten Seite der Leiterplatte befestigt. Beispielsweise kann es sich bei dem auf dem Dichtverschluss angeordneten ersten Magneten um einen Permanentmagneten handeln und bei dem zweiten Magneten um eine metallische Platte, die auf der der Fluidkanalöffnung abgewandten Seite der Leiterplatte angeordnet ist. Diese Ausführungsform hat den Vorteil eines einfachen Aufbaus, der bevorzugt auch von einem Bestückungsautomaten im Rahmen einer Bestückung der Leiterplatte bewerkstelligt werden kann.

Besonders bevorzugt sind der erste Magnet und der zweite Magnet der Überdruckventilanordnung der Leiterplatte derart angeordnet, dass die zwischen ihnen wirkende Magnetkraft einer Verschiebung des ersten Magneten hinaus aus einer zentralen Position entgegenwirkt. Damit wirken die beiden Magneten selbst-zentrierend und selbst-führend, was einer vorteilhaften Öffnungs- und Schließcharakteristik der Überdruckventilanordnung zu gute kommt. Insbesondere vermeidet die Überdruckventilanordnung so selbsttätig ein verkantetes Aufliegen des Dichtverschluss auf der Fluidkanalöffnung. Beispielsweise ist also der zweite Magnet auf der der Fluidkanal abgewandten Seite der Leiterplatte in dieser integriert angeordnet und oberhalb des zweiten Magneten der Dichtverschluss mitsamt des ersten Magneten auf der Magnetkanalöffnung.

Zur Erzielung der oben genannten vorteilhaften Wirkung ist es zweckmäßig, wenn der erste Magnet und der zweite Magnet einander zugewandte Flächen aufweisen, die jeweils möglichst zueinander zentriert und im Wesentlichen senkrecht zu einer gedachten vertikalen Achse angeordnet sind und vorzugsweise eine im Wesentlichen gleiche Form und Größe haben. Auf Grund der Magnetkraft zwischen dem ersten und dem zweiten Magneten wirkt diese Überdruckventilanordnung selbst-zentrierend, da die Magnetkraft einer Verschiebung des ersten Magneten hinaus aus einer gedachten projizierten Fläche parallel zur Fläche des zweiten Magneten entgegen wirkt, und zwar sowohl einer Verkippung des ersten Magneten, als auch einer horizontale Verschiebung.

Zur Erzielung eines kompakten Aufbaus der erfindungsgemäßen Leiterplatte ist es weiterhin förderlich, wenn der zweite Magnet in eine Aussparung der Leiterplatte eingebettet ist. Beispielsweise kann der zweite Magnet in dieser Aussparung im Sinne eines Inlays befestigt, insbesondere in diese eingeklebt sein.

Der erste Magnet ist bevorzugt auf dem Dichtverschluss aufgeklebt. Der zweite Magnet ist bevorzugt auf der der Fluidkanalöffnung abgewandten Seite der Leiterplatte aufgeklebt.

Ebenfalls ist es für einen kompakten Aufbau der Leiterplatte förderlich, wenn der erste Magnet und der zweite Magnet jeweils einen planen Körper bilden, dessen Breite wesentlich größer ist als dessen Höhe. Auf diese Weise lässt sich erreichen, dass die Überdruckventilanordnung eine flache Struktur aufweist, die sich, wenn überhaupt, kaum von übrigen Bauteilen auf der Leiterplatte abhebt. Beispielsweise hat der plane Körper eine Breite, die wenigstens zweimal so groß ist, bevorzugt wenigstens dreimal so groß wie die Höhe. Denkbar ist beispielsweise ein scheibenförmiger Körper, also ein Zylinder, mit einer Höhe von 2,5 mm und einem Durchmesser von 12 mm.

Bevorzugt liegt die gesamte Höhe der Überdruckventilanordnung unter 1 cm.

Um die Gesamthöhe der Überdruckventilanordnung möglichst gering zu halten, ist es bevorzugt, wenn der Dichtverschluss ein Dichtelement, wie einen O-Ring, aufweist, das im geschlossenen Zustand der Überdruckventilanordnung in Aussparungen der Leiterplatte eingebettet ist. Solche Aussparungen können beispielsweise solche sein, die normalerweise für elektrische Leiterbahnen auf der Oberfläche der Leiterplatte vorgesehen sind. Auch daran wird deutlich, dass die gesamte Überdruckventilanordnung keinen besonderen Aufwand erfordert, der weit über den üblichen Aufwand einer Bestückung einer Leiterplatte hinausgeht: eventuell vorgesehene Aussparungen auf der Oberfläche der Leiterplatte können mit Mitteln erzeugt werden, die ohnehin bereits zur Erzeugung der Aussparungen für Leiterbahnen vorhanden sind. Der erste Magnet kann beispielsweise bereits auf dem Dichtverschluss aufgeklebt sein. Der erste Magnet mit dem Dichtverschluss und der zweite Magnet können dann mit einem Bestückungsautomaten auf der Leiterplatte positioniert werden, so dass eine bestückte fertige Leiterplatte bereits mit der Überdruckventilanordnung im geschlossenen Zustand versehen ist.

Das Dichtelement des Dichtverschlusses kann beispielsweise ein O-Ring oder eine gestanzte Flachdichtung sein, grundsätzlich aber beliebig ausgestaltet sein, demnach auch von Der Form des O-Rings oder der gestanzten Flachdichtung abweichen.

Der zweite Magnet kann beispielsweise ein Inlay sein, beispielsweise aus Eisen oder Ferrit. Ein solches Inlay kann ein Leiterplattenhersteller gleich in die Leiterplatte einlaminieren.

Damit der erste Magnet und der Dichtverschluss im Falle eines starken Überdrucks im Fluidkanal nicht einfach von der Leiterplatte gelöst wird und die Magnetkraft nicht mehr ausreicht, den ersten Magneten samt des Dichtverschlusses nach Abfall des Überdrucks wieder zu positionieren, ist es zweckmäßig, wenn die Leiterplatte ein zusätzliches elektronisches Bauteil, wie ein passives elektronisches Bauteil, wie ein Widerstand, ein Kondensator oder eine Drahtbrücke oder ein aktives elektronisches Bauteil, wie ein Transistor oder ein integrierter Schaltkreis, umfasst, das derart angeordnet ist, dass es den ersten Magneten überbrückt und ausgebildet ist, zu verhindern, dass der Abstand zwischen dem ersten Magneten und dem zweiten Magneten im Falle eines Überdrucks einen Höchstabstand überschreitet. Vorteilhaft an dieser Ausführungsform ist, dass auch das zusätzliche elektronische Bauteil im Rahmen eines üblichen Bestückungsverfahrens mit einem Bestückungsautomaten installiert werden kann. Denkbar ist beispielsweise eine Anordnung, bei der ein ohmscher Widerstand den dem Dichtverschluss angeordneten ersten Magneten räumlich (im Gegensatz zu elektrisch) überbrückt und insofern mechanisch stabilisiert. Die Überbrückung des ersten Magneten dient vordergründig dem Verhindern des Überschreitens eines vorbestimmten Abstandes, nicht jedoch einer Führung des Dichtverschlusses während eines auf Grund eines Überdrucks im Fluidkanal hervorgerufenen Hubs des Dichtverschlusses. Die Führung des beweglichen Magneten erfolgt bevorzugt, wie bereits erläutert, durch die Magnetkraft selbst.

In einer weiteren bevorzugten Ausführungsform sind der erste Magnet und der zweite Magnet derart angeordnet, dass sie im geschlossenen Zustand der Überdruckventilanordnung in keinem unmittelbaren Kontakt zu einem Fluid im Fluidkanal stehen. Diese Ausführungsform hat den Vorteil, dass das Material des ersten und des zweiten Magneten nicht auf die chemische Zusammensetzung eines im Fluidkanal befindlichen Fluids angepasst werden muss. Beispielsweise ist es bevorzugt, dass der erste Magnet zumindest auf einer dem Fluidkanal zugewandten Seite mit Epoxid beschichtet ist, beispielsweise mit einer 10 µm dicken Expoxidschicht.

Bei der erfindungsgemäßen Leiterplatte handelt es sich vorzugsweise um eine mehrschichtige Leiterplatte. Bei einer mehrschichtigen Leiterplatte lassen sich Fluidkanäle vergleichsweise einfach realisieren, beispielsweise so, wie es in der Offenlegungsschrift DE 197 397 22 A1 beschrieben ist. Alle Schichten der mehrschichtigen Leiterplatte sind vorzugsweise herkömmliche glasfaserverstärkte Epoxidharzleiterplatten, beispielsweise solche mit einer Materialkennung FR4.

Einen zweiten Aspekt der vorliegenden Erfindung bildet ein Insufflator. Insufflatoren werden in der Laparoskopie (der endoskopischen Abdominalchirurgie) eingesetzt und dienen dazu, einem Operateur durch ein Endoskop eine freie Sicht auf ein Operationsfeld im Abdomen zu verschaffen, indem in das Abdomen Kohlendioxid (CO₂) eingeleitet wird. Hierzu wird über eine sogenannte Verres-Kanüle oder einen Trokar CO₂-Gas mit einem maximalen Druck von 30 mm_{Hg} in das Abdomen insuffliert. Durch den steigenden Innendruck wird die Bauchdecke (Peritoneum) angehoben und im Abdomen entsteht der gewünschte Hohlraum, der die endoskopische Betrachtung des Operationsfeldes erlaubt.

Der Insufflator des zweiten Aspektes der vorliegenden Erfindung weist eine Leiterplatte des ersten Aspektes der Erfindung auf. Ein wesentlicher Vorteil des Insufflators ist seine besonders sichere und zuverlässige Betriebsweise, die vor allem auf die kostengünstige, kompakte sowie betriebssichere Überdruckventilanordnung mit hervorragender Öffnungs- und Schließcharakteristik der Leiterplatte zurückgeht.

Ein weiterer Vorteil des erfindungsgemäßen Insufflators besteht darin, dass die Leiterplatte mit dem wenigstens einen Fluidkanal im übrigen solchen Leiterplatten entspricht, wie sie in bekannten Geräten für elektronischen Komponente verwendet werden, so dass die elektronischen Komponenten über die Leiterplatte mit dem ansonsten separat realisierten fluidischen Elementen eines Insufflators zu einer Baugruppe zusammengefasst werden.

Beispielsweise ist der Insufflator des zweiten Aspektes der vorliegenden Erfindung ausgestaltet, mit einem Zuleitungsanschluss und einem Ableitungsanschluss und einer zwischen diesen Anschlüssen angeordneten Druck- und Strömungsmesseinrichtung zum Bestimmen eines am Ableitungsanschluss anliegenden Gasdrucks und zum Bestimmen von einen am Ableitungsanschluss anliegenden Gasvolumenstrom charakterisierenden Messgrößen, die die Leiterplatte umfasst, wobei
- der Fluidkanal der Leiterplatte an seinem Eingang mit dem Zuleitungsanschluss und an seinem Ausgang mit dem Ableitungsanschluss verbunden ist und
- auf der Leiterplatte Druckmesssensoren sowie elektronische Bauelemente zur Beschaltung der Druckmesssensoren angeordnet sind, von denen die Druckmesssensoren jeweils durch eine entsprechende Öffnung in einer Leiterplattenschicht mit dem Fluidkanal in unmittelbarer Verbindung stehen und ausgebildet sind, einen den am Ort der jeweiligen Öffnung herrschenden statischen Druck repräsentierenden Ausgangswert zu liefern.

In einer bevorzugten Ausführungsform des Insufflators ist der Fluidkanal von einem Hohlraum in der Leiterplatte gebildet, die derart geformt ist, dass er einen Abschnitt aufweist, der als Strömungsdrossel wirkt und eine Volumenstrommessung nach dem Prinzip des Pneumotachographen erlaubt.

Vorzugsweise ist in dieser Ausführungsform am Eingang und am Ausgang des als Strömungsdrossel wirkenden Abschnitts des Hohlraums jeweils ein Drucksensor angeordnet, die mit elektronischen Komponenten verbunden sind und die ausgebildet sind, eine Differenz zwischen dem statischen Druck am Eingang des als Strömungsdrossel wirkenden Abschnitts des Hohlraums und dem statischen Druck am Ausgang des als Strömungsdrossel wirkenden Abschnitts des Hohlraums zu bestimmen. Der Insufflator ist demnach bevorzugt dazu ausgebildet, eine Strömungsregelung durch eine Druckregelung zu implementieren. Dazu ist der Insufflator in einer Ausführungsform ausgebildet, nach einem Niederdruckprinzip zu verfahren, bei dem der Insufflationsdruck gleich einem Solldruck ist, der in der Regel einem theoretischen maximalen Druck im Abdomen entspricht. Aufgrund eines Druckabfalls über einen Einlass und entlang einer Wandung eines zum Abdomen führenden Schlauches herrscht im Abdomen tatsächlich ein niedrigerer Druck als der Solldruck. Dieses Verfahren stellt meistens ein kontinuierliches Verfahren dar.

In einer anderen Ausführungsform ist der Insufflator ausgebildet, nach einem Überdruckprinzip zu arbeiten, bei dem der Insufflationsdruck stufenweise höher als der Solldruck eingestellt wird, wobei hier eine Messung eines intraabdominellen Drucks zyklisch in Pausen erfolgt, in denen der Insufflationsdruck und der Volumenstrom auf einen Betrag von jeweils etwa Null eingestellt werden. Dieses Überdruckverfahren stellt zumeist ein intermittierendes Verfahren dar.

In einer bevorzugten Ausführungsform ist der Insufflator ausgebildet, das Gas gemäß dem aus der Veröffentlichung der europäischen Patentanmeldung EP 1352669 A1 quasikontinuierlichen Verfahren in den Körper des Patienten einzuleiten. Auf diese Veröffentlichung, insbesondere auf die Ausführungsbeispiele gemäß den Fig. 4 und 5, wird hiermit explizit verwiesen.

Nachfolgend werden weitere Vorteile der vorliegenden Erfindung mit Bezug auf die Zeichnung erläutert. Darin zeigen:
- Fig. 1:: eine schematische Darstellung einer Ausführungsform der erfindungsgemäßen Leiterplatte mit einer Überdruckventilanordnung,
- Fig. 2:: ein Kraftwegdiagramm,
- Fig. 3:: eine schematische Darstellung des prinzipiellen Aufbaus einer Ausführungsform Überdruckventilanordnung der Leiterplatte,
- Fig. 4:: eine Fotographie einer auf einer Leiterplatte mit einem Fluidkanal angeordneten Überdruckventilanordnung,
- Fig. 5:: ein weiteres Kraftwegdiagramm,
- Fig. 6:: eine schematische Darstellung eines Querschnitts durch einen erfindungsgemäßen Insufflator,
- Fig. 7:: eine schematische Darstellung einer Aufsicht auf eine Leiterplatte des erfindungsgemäßen Insufflators.

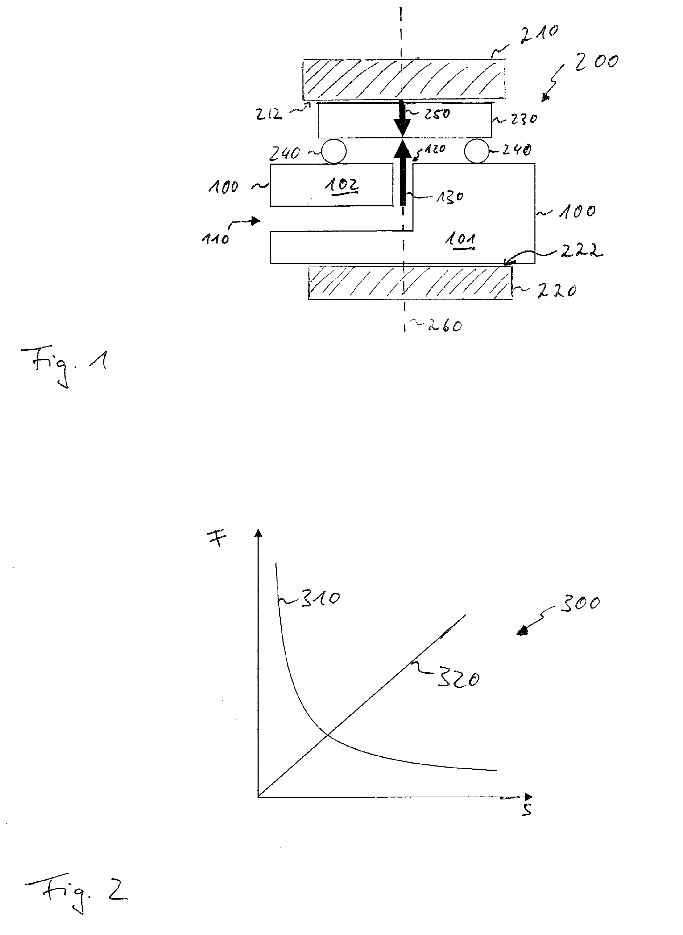

Fig. 1 zeigt in schematischer Darstellung einen Querschnitt einer erfindungsgemäßen Leiterplatte 100 mit Überdruckventilanordnung 200. Die Leiterplatte 100 ist eine mehrschichtige Leiterplatte mit wenigstens zwei Schichten, der ersten Schicht 101 und der zweiten Schicht 102.

Die Leiterplatte 100 weist in ihrem Inneren wenigstens einen Fluidkanal 110 auf, welcher ein Fluid, wie beispielsweise CO₂ oder H₂O, führen kann. Der in Fig. 1 angedeutete Fluidkanal 110 kann zu einem im Inneren der Leiterplatte 100 angeordneten Fluidkanalsystem führen, welches aber in Fig. 1 nicht dargestellt ist. Die Leiterplatte 100 weist auf einer Oberfläche eine Fluidkanalöffnung 120 auf, welche zum Fluidkanal 110 führt. Über der Fluidkanalöffnung 120 ist ein Dichtverschluss 230 mit einem Dichtelement 240 in Gestalt eines O-Rings angeordnet. Auf dem Dichtverschluss 230 ist ein erster Magnet 210 befestigt, beispielsweise angeklebt. Auf einer der Fluidkanalöffnung 120 abgewandten Seite der Leiterplatte 100 ist ein zweiter Magnet 220 befestigt, beispielsweise ebenfalls angeklebt. Der erste Magnet 210, der zweite Magnet 220 und der Dichtverschluss 230 mitsamt des Dichtelements 240 sind Teil der Überdruckventilanordnung 200.

Zwischen dem ersten Magneten 210 und dem zweiten Magneten 220 wirkt eine mit dem Pfeil 250 angedeutete Magnetkraft, so dass der Dichtverschluss 230 auf die Fluidkanalöffnung 120 gepresst wird und diese abdichtet. Entsteht im Fluidkanal 110 ein Druck, so wirkt eine durch den Pfeil 130 angedeutete Druckkraft in einer der Magnetkraft 250 entgegengesetzten Richtung. Übersteigt die Druckkraft 130 die Magnetkraft 250, so öffnet die Überdruckventilanordnung 200 und ein Überdruck im Fluidkanal 110 wird abgebaut.

Der erste Magnet 210 kann beispielsweise ein Permanentmagnet sein. Der zweite Magnet 220 kann beispielsweise eine metallische Platte sein. Die Begriffe erster Magnet und zweiter Magnet sind demnach in einem technisch funktionalen Sinne zu verstehen, dass zwischen Ihnen eine Magnetkraft wirkt. Entgegen der Darstellung in Fig. 1 kann der zweite Magnet in der ersten Schicht 101 der Leiterplatte 100 integriert sein.

Bei der in Fig. 1 dargestellten Ausführungsform der erfindungsgemäßen Leiterplatte weisen der erste Magnet 210 und der zweite Magnet 220 einander zugewandte Flächen 212, 222 auf, die jeweils im Wesentlichen zentriert und im Wesentlichen senkrecht zu einer gedachten vertikalen Achse 260 angeordnet sind und im Wesentlichen die gleiche Form haben. Diese Ausgestaltung hat den Vorteil, dass der erste Magnet 210 und der zweite Magnet 220 eine Magnetkraft 250 erzeugen, die einer Verschiebung des ersten Magneten 210 hinaus aus der gezeigten zentralen Position entgegenwirkt. Die Magnetkraft 250 wirkt also sowohl einer Verkippung des ersten Magneten 210 entgegen, also einem Zustand, bei dem die Fläche 212 des ersten Magneten 210 nicht senkrecht zur gedachten Achse 260 liegt, als auch einer Verschiebung des ersten Magneten 210 entlang der Horizontalen nach links oder rechts.

Ebenfalls ist in Figur 1 angedeutet, dass der erste Magnet 210 und der zweite Magnet 220 jeweils einen planen Körper haben, bei dem die Breite wesentlich größer ist als die Höhe. Dies kommt einem kompakten Aufbau der Leiterplatte 100 zugute.

Auch zeigt Fig. 1, dass der erste Magnet 210 und der zweite Magnet 220 derart angeordnet sind, dass sie in keinen unmittelbaren Kontakt zu einem Fluid im Fluidkanal 110 stehen. Dies hat den Vorteil, dass das Material des ersten Magneten 210 und das Material des zweiten Magneten 220 nicht auf die chemische Zusammensetzung eines im Fluidkanal 110 befindlichen Fluids angepasst werden muss.

Ein Vorteil der Überdruckventilanordnung 200 gegenüber einem mit einer Feder betriebenen Ventil ist in Figur 2 angedeutet. Figur 2 zeigt ein Kraft-Weg-Diagramm für eine Magnetkraft zwischen dem ersten und den zweiten Magneten 210, 220 und für den Fall, dass eine Dichtung nicht durch eine Magnetkraft, sondern durch eine Federkraft auf eine Öffnung gepresst wird. Im Kraft-Weg-Diagramm 300 ist auf der Ordinate die Kraft aufgetragen und auf der Abszisse der Weg. Der Verlauf 310 stellt den Verlauf einer Magnetkraft dar und der Verlauf 320 den Verlauf einer mechanischen Federkraft.

Ein erster Vorteil der mittels Magnetkraft betriebenen Überdruckventilanordnung besteht darin, dass die Magnetkraft mit sinkendem Abstand zunimmt, so dass die in Figur 1 dargestellte Kraft 250 im geschlossenen Zustand der Überdruckventilanordnung 200 ein Maximum annimmt. Dahingegen muss zum Aufbau einer hohen Kraft mittels einer Feder eine gewisse Strecke zur Verfügung stehen, was sich im Ergebnis vor allem in einem größeren und aufwendigeren Aufbau äußert. Öffnet sich das Ventil aufgrund eines Überdrucks, so wird im Falle vom Magneten der Überdruck schneller abgebaut, da die Schließkraft mit wachsendem Abstand abnimmt und das magnetbetriebene Ventil insofern schneller öffnet. Dahingegen steigt die Schließkraft bei einem mit einer Feder betriebenen Ventil mit steigendem Abstand.

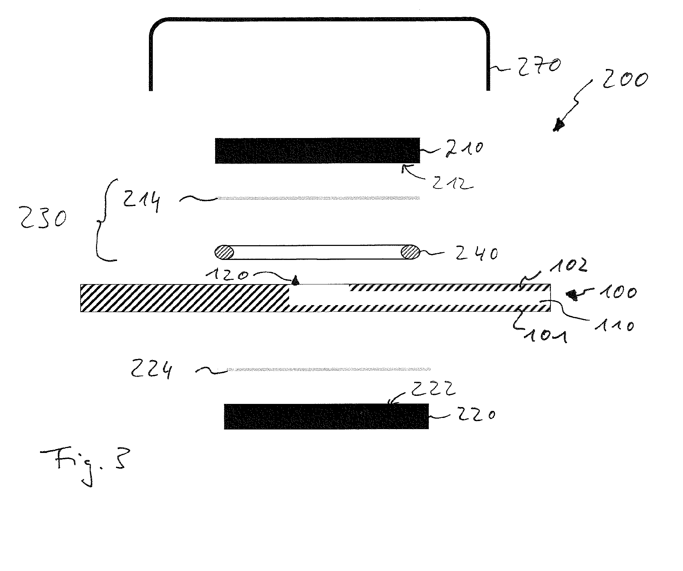

Figur 3 zeigt in schematischer Darstellung einen Querschnitt durch die Leiterplatte 100 mit der Überdruckventilanordnung 200. Der Aufbau aus Figur 3 stimmt grundsätzlich mit dem in Figur 1 dargestellten Aufbau überein, auch zeigt Figur 3 zusätzlich eine bevorzugte Variante des Dichtverschlusses 230. Dabei wird der Dichtverschluss 230 durch eine erste Klebeschicht 214 und dem Dichtelement 240 in Gestalt eines O-Rings gebildet. Der O-Ring 240 wird bei der Installation der Überdruckventilanordnung 200 auf die erste Klebeschicht 214 unten aufgeklebt und der erste Magnet 210 von oben. Die erste Klebeschicht 214 fungiert damit auch als Dichtfolie.

Der zweite Magnet 220 wird bevorzugt mittels einer zweiten Klebeschicht 224 auf die der Fluidkanalöffnung 120 abgewandten Seite der Leiterplatte 100 angeklebt. Figur 3 verdeutlicht somit wieder den kompakten Aufbau der Überdruckventilanordnung 200. Um zu vermeiden, dass sich der erste Magnet 210 mit dem angeklebten Dichtverschluss 230 im Falle eines Überdrucks im Fluidkanal 110 zu weit von dem angeklebten zweiten Magneten 220 entfernt, ist eine Haltevorrichtung 270 vorgesehen, die den ersten Magneten 210 überbrückt. Diese Haltevorrichtung 270 ist bevorzugt ein elektronisches Bauteil, wie ein passives elektronisches Bauteil, wie ein ohmscher Widerstand oder ein Kondensator, oder ein aktives elektronisches Bauteil, wie ein Transistor oder ein integrierter Schaltkreis. Dies hat den Vorteil, dass auch die Haltevorrichtung 270 im Zuge eines Bestückungsverfahrens mittels eines Bestückungsautomaten auf der Leiterplatte 100 installiert werden kann.

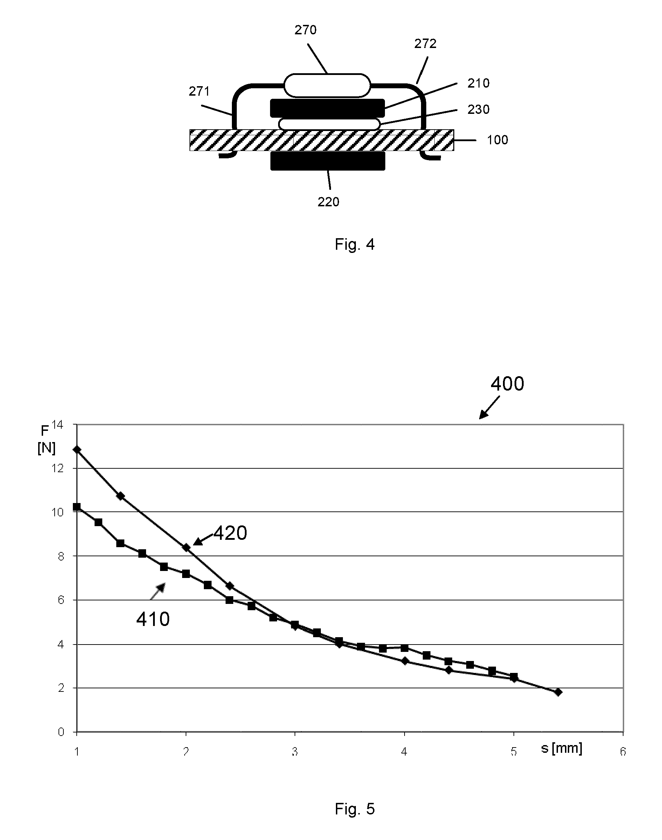

Figur 4 zeigt eine Fotografie einer realisierten Leiterplatte 100 mit einer Überdruckventilanordnung, dessen Aufbau im Wesentlichen dem in Figur 3 schematisch dargestellten Aufbau entspricht. Als Haltevorrichtung 270 wurde hier ein ohmscher Widerstand eingesetzt, der den ersten Magneten 210 und den Dichtverschluss 230 mithilfe seiner Lötarme 271, 272 überbrückt und in soweit verhindert, dass sich der erste Magnet 210 mitsamt dem Dichtverschluss 230 im Falle eines Überdrucks im in Figur 4 nicht sichtbaren Fluidkanal zu weit von dem zweiten Magneten 220 unterhalb der Leiterplatte 100 entfernt.

Der erste Magnet 210 und der zweite Magnet 220 aus Figur 4 bestehen aus einer Neodym Eisen Bor Legierung des Grades N35 und haben jeweils einen Durchmesser von 12 mm und eine Höhe von 2,5 mm. Die in Figur 4 dargestellte Überdruckventilanordnung öffnet etwa bei einem Druck im Fluidkanal zwischen 290 bis 310 mBar.

Dieser Druckbereich kann aber, wie bereits an andere Stelle ausführlicher erläutert, beliebig eingestellt werden, beispielsweise durch Wahl eines bestimmten Magnetmaterials und durch Veränderung der Abmessungen des ersten Magneten 210 und des zweiten Magneten 220. Grundsätzlich ist es vorteilhaft, dass das Material des Dichtverschlusses, insbesondere des Dichtelements in Gestalt eines O-Rings, so gewählt wird, dass ein Anhaften auf der Leiterplatte 100 vermieden wird, so dass die Überdruckventilanordnung in der Tat bei einem jeweils gleichen Überdruck im Fluidkanal 110 öffnet.

Die in Figur 4 dargestellte Überdruckventilanordnung wurde für einen Überdruck von etwa 300 mBar entworfen. Der Durchmesser des Dichtverschlusses 230 beträgt etwa 10 mm. Insgesamt zählt die in Figur 4 dargestellte Überdruckventilanordnung geschlossenen Zustand eine Magnetkraft von etwa 2,4 N. Demnach öffnet sich die Überdruckventilanordnung bei einem Überdruck dem Fluidkanal, der eine Druckkraft größer als 2,4 N bewirkt.

Im Anschluss zeigt Figur 5 ein weiteres Kraft-Weg-Diagramm 400, das zum einen einen gemessenen Kraft-Weg-Verlauf 410 darstellt und zum anderen im Vergleich dazu einen simulierten Kraft-Weg-Verlauf 420. Sowohl die Messung als auch die Simulation beziehen sich auf die in Figur 4 dargestellte Anordnung. Auf der Ordinate ist wieder die Kraft aufgetragen und auf der Abszisse der Abstand zwischen dem ersten Magneten 210 und dem zweiten Magneten 220 aus Figur 4. Die Simulationsergebnisse basieren weitestgehend auf Schätzungen und Tabellenwerten. Durchgeführt wurde diese Simulation mit dem Programm COMSOL 3.4. Dabei wurde der Einfluss eines Materials zwischen dem ersten Magneten und dem zweiten Magneten nicht beachtet. Sowohl die Simulationsergebnisse 420 als auch die Messergebnisse 410 zeigen, dass für eine Kraft von etwa 2,4 N eine Distanz zwischen dem ersten Magneten 210 und dem zweiten Magneten 220 von etwa 4,5 mm angemessen ist. Ist weder das Dichtelement 240 noch der zweite Magnet 220 in die Leiterplatte eingebettet, so ergibt sich die eben erwähnte Distanz schlicht durch Aufsummieren der Dicken der zweiten Klebeschicht 224, der Leiterplatte 100, des Dichtelements in Gestalt eines O-Rings 240 und der ersten Klebeschicht 214.

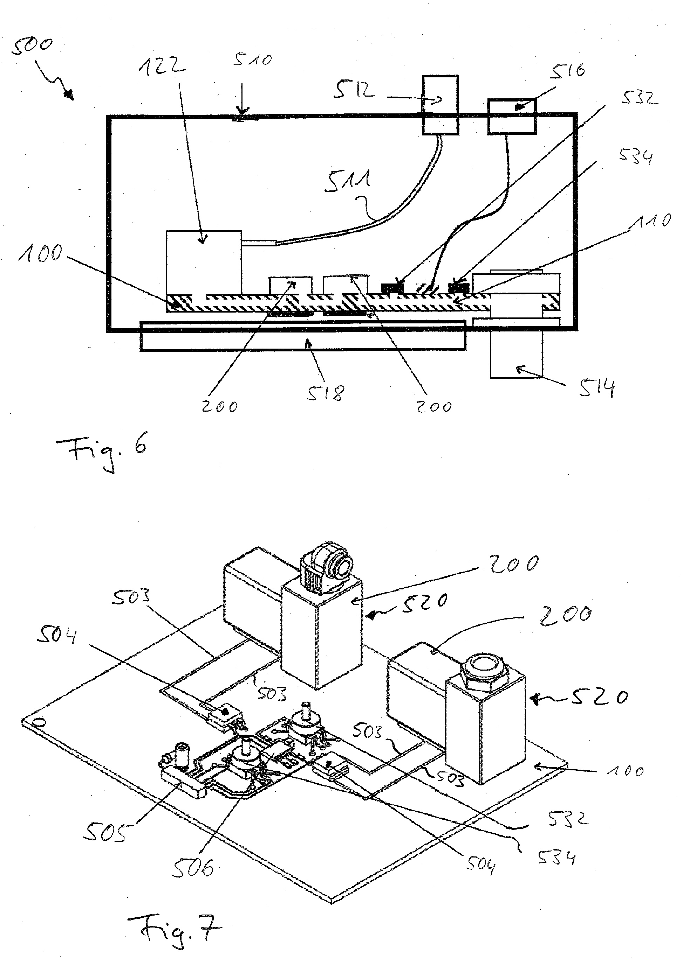

In Figur 6 ist schematisch ein Gehäuse 510 eines erfindungsgemäßen Insufflators 500 angedeutet, an welchem ein CO₂-Anschluss an eine Quelle für gasförmiges CO₂ vorgesehen ist sowie ein Ableitungsanschluss 514, an dem ein Schlauch zum Einführen in das Abdomen eines Patienten anzuschließen ist. Außerdem weist der Insufflator 500 einen elektrischen Netzanschluss 516 sowie ein Bedienpanel 518 auf.

Kernstück des Insufflators 500 ist die Leiterplatte 100, auf die ein Zuleitungsanschluss 122 und der Ableitungsanschluss 514 gasdicht aufgeklebt sind. Der Zuleitungsanschluss 122 ist mit den CO₂-Anschluss 512 über einen entsprechenden Schlauch 511 verbunden.

Die Leiterplatte 100 aus Figur 6 ist mehrschichtig und weist in ihrem Inneren Hohlräume auf, die mehrere Fluidkanäle 110 bilden. Der Zuleitungsanschluss 122 und der Ableitungsanschluss 514 sind mit dem Fluidkanal 110 fluidverbunden. Außerdem sind auf der Außenseite der Leiterplatte 100 elektrische und elektronische Bauteile des Insufflators 500 montiert sowie wenigstens eine Überdruckventilanordnung 200 vorgesehen. Dargestellt sind zwei Überdruckventilanordnungen 200, wobei eine einzige ebenfalls die beschriebene Vorteile für den Insufflator erzielen kann.

Die Überdruckventilanordnungen 200 sind ebenfalls gasdicht auf der Leiterplatte 100 aufgeklebt. Die Leiterplatte 100 des Insufflators 500 umfasst demnach zwei Überdruckventilanordnungen 200, die jeweils zwei Fluidkanalöffnungen absichern.

Außerdem sind Drucksensoren 532, 534 vorgesehen, welche als Differenzdrucksensoren ausgestaltet sind und rückwertig auf der Leiterplatte 100 gasdicht aufgeklebt sind.

Die verschiedenen Abschnitte des Fluidkanals 110 im Inneren der Leiterplatte 100 haben eine lichte Höhe von etwa 1 mm und ein licht Breite zwischen 1 mm und 8 mm.

Der Insufflator 500 erlaubt einen Gasvolumenstrom von CO₂ von bis zu 44 l/min. Er ist für einen Intraabdominaldruck von etwa 1 bis 30 mm_{Hg} ausgelegt. Durch die Überdruckventilanordnungen 200 ist ein intermittierender Gasfluss möglich.

Figur 7 zeigt in schematischer Darstellung nochmals das Kernstück des Insufflators 500 aus Figur 6, nämlich die mehrschichtige Leiterplatte 100 mit dem darauf montierten elektronischen und fluidischen Bauelementen, die für den beispielhaft dargestellten Insufflator 500 erforderlich sind.

Dies sind die Überdruckventile 200, leistungselektronische Bauelemente 504, welche mit den Überdruckventilen 200 über Leiterbahnen 503 verbunden sind, die Drucksensoren 532 und 534, einen Steckverbinder 505 zum Verbinden mit den in Figur 7 nicht dargestellten Netzanschluss 516, eine Sensorsignalaufbereitung, ein Mikrocontroller 506, Tasten und Anzeigen.

Die Fluidkanäle im Inneren der mehrschichtigen Leiterplatte 100 sind in Figur 7 nicht zu erkennen. Sie stellen die fluidische Verbindung zwischen dem Zuleitungsanschluss 122 und dem Ableitungsanschluss 514 sowie den Überdruckventilen 200 und den Drucksensoren 532 und 534 dar, die als pneumatische (fluidische) Bauelemente ebenfalls auf der Leiterplatte 100 befestigt sind. Die fluidischen Bauelemente sind dabei so angeordnet, dass sie sowohl fluidischen Kontakt zu den im Inneren der Leiterplatte 100 angeordneten Fluidkanälen als auch elektrischen Kontakt zu den elektrischen und elektronischen Bauelementen 504 und 506 des Insufflators 500 haben. Diese doppelte Nutzung der Leiterplatte 100 ermöglicht einen kompakten und preiswerten Aufbau eines Insufflators.

### Bezugszeichenliste

- 100: Leiterplatte
- 101: erste Schicht der Leiterplatte
- 102: zweite Schicht der Leiterplatte
- 110: Fluidkanal
- 120: Fluidkanalöffnung
- 122: Zuleitungsanschluss
- 130: Druckkraft

- 200: Überdruckventilanordnung
- 210: erster Magnet
- 212: dem Fluidkanal zugewandte Fläche des ersten Magneten
- 214: erste Klebeschicht
- 220: zweiter Magnet
- 222: dem Fluidkanal zugewandte Fläche des zweiten Magneten
- 224: zweite Klebeschicht
- 230: Dichtverschluss
- 240: Dichtelement
- 250: Magnetkraft oder Schließkraft
- 260: gedachte vertikale Achse
- 270: Haltevorrichtung
- 271,272: Lötarme

- 300: Kraft-Weg-Diagramm
- 310: Magnetkraft in Abhängigkeit einer Strecke
- 320: Federkraft in Abhängigkeit einer

- 400: weiteres Kraft-Weg-Diagramm
- 410: gemessener Kraft-Weg-Verlauf
- 420: simulierter Kraft-Weg-Verlauf

- 500: Insufflator
- 503: Leiterbahnen
- 504: Leistungselektronische Bauelemente
- 505: Netzanschluss
- 506: Mikrocontroller
- 510: Gehäuse des Insufflators
- 511: Schlauch
- 512: CO₂-Anschluss
- 514: Ableitungsanschluss
- 516: Netzanschluss
- 518: Bedienpanel
- 520: Gehäuse der Überdruckventilanordnung
- 532,534: Drucksensoren

## Patentansprüche

1. Leiterplatte (100) mit wenigstens einem im Inneren der Leiterplatte (100) angeordneten Fluidkanal (110), aufweisend:
- eine auf einer Oberfläche der Leiterplatte (100) angeordnete Fluidkanalöffnung (120), welche zum Fluidkanal (110) führt, und
- eine mehrstückige Überdruckventilanordnung (200) zum Abbauen eines Überdrucks im Fluidkanal (110), welche einen ersten Magneten (210), einen zweiten Magneten (220) und einen Dichtverschluss (230) umfasst, wobei
- der erste Magnet (210) und der zweite Magnet (220) derart angeordnet sind, dass diese den Dichtverschluss (230) durch eine von dem ersten Magneten (210) und von dem zweiten Magneten (220) bewirkte Magnetkraft (250) auf die Fluidkanalöffnung (120) pressen, und wobei
- die Überdruckventilanordnung (200) so ausgestaltet ist, dass sie nach einem Überdruck im Fluidkanal (110) selbständig wieder in den geschlossenen Zustand übergeht.

2. Leiterplatte (100) nach Anspruch 1, bei der der erste Magnet (210) auf dem Dichtverschluss (230) und der zweite Magnet (220) auf einer der Fluidkanalöffnung (120) abgewandten Seite der Leiterplatte (100) befestigt sind.

3. Leiterplatte (100) nach Anspruch 1 oder 2, bei der der erste Magnet (210) und der zweite Magnet (220) derart angeordnet sind, dass die Magnetkraft (250) einer Verschiebung des ersten Magneten (210) hinaus aus einer zentralen Position entgegenwirkt.

4. Leiterplatte (100) nach einem der vorstehenden Ansprüche, bei der erste Magnet (210) und der zweite Magnet (220) einander zugewandte Flächen (212, 222) aufweisen, die jeweils im Wesentlichen zentriert und im Wesentlichen senkrecht zu einer gedachten vertikalen Achse (260) angeordnet sind und im Wesentlichen die gleiche Form haben.

5. Leiterplatte (100) nach einem der vorstehenden Ansprüche, bei der der zweite Magnet (220) in einer Aussparung der Leiterplatte (100) eingebettet ist.

6. Leiterplatte (100) nach einem der vorstehenden Ansprüche, bei der der erste Magnet (210) und der zweite Magnet (220) jeweils einen planen Körper haben, bei dem die Breite wesentlich größer ist als die Höhe.

7. Leiterplatte (100) nach einem der vorstehenden Ansprüche, bei der der Dichtverschluss (230) ein Dichtelement (240) aufweist, das im geschlossenen Zustand der Überdruckventilanordnung (200) in Aussparungen der Leiterplatte (100) eingebettet ist.

8. Leiterplatte (100) nach einem der vorstehenden Ansprüche, bei der der Dichtverschluss (230) einen O-Ring als Dichtelement (240) umfasst.

9. Leiterplatte (100) nach einem der vorstehenden Ansprüche, umfassend ein elektronisches Bauteil (270), wie ein passives elektronisches Bauteil, wie ein Widerstand oder ein Kondensator, oder wie ein aktives elektronisches Bauteil, wie ein Transistor oder ein integrierter Schaltkreis, das derart angeordnet ist, dass es den ersten Magneten (210) überbrückt und ausgebildet ist, zu verhindern, dass der Abstand zwischen dem ersten Magneten (210) und dem zweiten Magneten (220) im Falle eines Überdrucks einen Höchstabstand überschreitet.

10. Leiterplatte (100) nach einem der vorstehenden Ansprüche, bei der erste Magnet (210) und der zweite Magnet (220) derart angeordnet sind, dass sie im geschlossenen Zustand der Überdruckventilanordnung (200) in keinem unmittelbaren Kontakt zu einem Fluid im Fluidkanal (110) stehen.

11. Leiterplatte (100) nach einem der vorstehenden Ansprüche, wobei die Leiterplatte (100) eine mehrschichtige Leiterplatte ist.

12. Insufflator (500) mit einer Leiterplatte (100) nach einem der vorstehenden Ansprüche.

13. Insufflator (500) nach Anspruch 12, mit einem Zuleitungsanschluss (122) und einem Ableitungsanschluss (514) und einer zwischen diesen Anschlüssen angeordneten Druck- und Strömungsmesseinrichtung zum Bestimmen eines am Ableitungsanschluss (514) anliegenden Gasdrucks und zum Bestimmen von einen am Ableitungsanschluss (514) anliegenden Gasvolumenstrom charakterisierenden Messgrößen, die die Leiterplatte (100) umfasst, wobei
- der Fluidkanal (110) der Leiterplatte (100) an seinem Eingang mit dem Zuleitungsanschluss (122) und an seinem Ausgang mit dem Ableitungsanschluss (514) verbunden ist und
- auf der Leiterplatte (100) Druckmesssensoren (532, 534) sowie elektronische Bauelemente (504, 506) zur Beschaltung der Druckmesssensoren (532, 534) angeordnet sind, von denen die Druckmesssensoren (532, 534) jeweils durch eine entsprechende Öffnung in einer Leiterplattenschicht mit dem Fluidkanal (110) in unmittelbarer Verbindung stehen und ausgebildet sind, einen den am Ort der jeweiligen Öffnung herrschenden statischen Druck repräsentierenden Ausgangswert zu liefern.

14. Insufflator (500) nach Anspruch 13, bei dem der Fluidkanal (110) von einem Hohlraum in der Leiterplatte (100) gebildet ist, der derart geformt ist, dass er einen Abschnitt aufweist, der als Strömungsdrossel wirkt und eine Volumenstrommessung nach dem Prinzip des Pneumotachographen erlaubt.

15. Insufflator (500) nach Anspruch 14, bei dem am Eingang und am Ausgang des als Strömungsdrossel wirkenden Abschnitts des Hohlraums jeweils ein Drucksensor (532, 534) angeordnet ist, die mit elektronischen Komponenten (504, 506) verbunden sind und die ausgebildet sind, eine Differenz zwischen dem statischen Druck am Eingang des als Strömungsdrossel wirkenden Abschnitts des Hohlraums und dem statischen Druck am Ausgang des als Strömungsdrossel wirkenden Abschnitts des Hohlraums zu bestimmen.

## Claims

1. Circuit board (100) having at least one fluid passage (110) arranged inside the circuit board (100), comprising:
- a fluid passage opening (120), which is arranged on a surface of the circuit board (100) and leads to the fluid passage (110), and
- a multi-part pressure-relief valve arrangement (200) for reducing excess pressure in the fluid passage (110), which arrangement comprises a first magnet (210), a second magnet (220) and a sealing closure (230), wherein
- the first magnet (210) and the second magnet (220) are arranged such that they press the sealing closure (230) onto the fluid passage opening (120) by a magnetic force (250) exerted by the first magnet (210) and by the second magnet (220), and wherein
- the pressure-relief valve arrangement (200) is constructed such that it reverts to the closed state independently after excess pressure in the fluid passage (110).

2. Circuit board (100) according to claim 1, in which the first magnet (210) is fixed to the sealing closure (230) and the second magnet (220) is fixed to a side of the circuit board (100) facing away from the fluid passage opening (120).

3. Circuit board (100) according to either claim 1 or claim 2, in which the first magnet (210) and the second magnet (220) are arranged such that the magnetic force (250) counteracts a displacement of the first magnet (210) out of a central position.

4. Circuit board (100) according to any of the preceding claims, in which the first magnet (210) and the second magnet (220) have faces (212, 222) which face one another, are each arranged substantially centrally and substantially perpendicularly to an imaginary vertical axis (260) and have substantially the same shape.

5. Circuit board (100) according to any of the preceding claims, in which the second magnet (220) is embedded in a recess in the circuit board (100).

6. Circuit board (100) according to any of the preceding claims, in which the first magnet (210) and the second magnet (220) each have a planar body, of which the width is substantially greater than the height.

7. Circuit board (100) according to any of the preceding claims, in which the sealing closure (230) has a sealing element (240) which, when the pressure-relief valve arrangement (200) is closed, is embedded in recesses in the circuit board (100).

8. Circuit board (100) according to any of the preceding claims, in which the sealing closure (230) comprises an O-ring as the sealing element (240).

9. Circuit board (100) according to any of the preceding claims, comprising an electronic component (270) such as a passive electronic component, such as a resistor or a capacitor, or such as an active electronic component, such as a transistor or an integrated circuit, which is arranged such that it bridges over the first magnet (210) and is designed to prevent the spacing between the first magnet (210) and the second magnet (220) exceeding a maximum spacing in the event of excess pressure.

10. Circuit board (100) according to any of the preceding claims, in which the first magnet (210) and the second magnet (220) are arranged such that, when the pressure-relief valve arrangement (200) is closed, they are not in direct contact with a fluid in the fluid passage (110).

11. Circuit board (100) according to any of the preceding claims, wherein the circuit board (100) is a multi-layer circuit board.

12. Insufflator (500) comprising a circuit board (100) according to any of the preceding claims.

13. Insufflator (500) according to claim 12, comprising a supply connection (122) and a discharge connection (514) and a pressure and flow measuring device which is arranged between said connections and is intended for determining a gas pressure applied at the discharge connection (514) and for determining parameters which are characteristic of a gas volume flow rate at the discharge connection (514), which device comprises the circuit board (100), wherein
- the fluid passage (110) of the circuit board (100) is connected at its inlet to the supply connection (122) and at its outlet to the discharge connection (514), and
- pressure measuring sensors (532, 534) and electronic components (504, 506) for connecting the pressure measuring sensors (532, 534) are arranged on the circuit board (100), the pressure measuring sensors (532, 534) of which are each directly connected to the fluid passage (110) through a corresponding opening in a circuit board layer and are designed to deliver an output value representing the static pressure prevailing at the location of the respective opening.

14. Insufflator (500) according to claim 13, in which the fluid passage (110) is formed by a cavity in the circuit board (100) which is shaped such that it has a portion that acts as a flow throttle and allows a volume flow measurement according to the principle of the pneumotachograph.

15. Insufflator (500) according to claim 14, in which at the inlet and at the outlet of the portion of the cavity acting as the flow throttle, a respective pressure sensor (532, 534) is arranged, is connected to electronic components (504, 506) and is designed to determine a differential between the static pressure at the inlet of the portion of the cavity acting as the flow throttle and the static pressure at the outlet of the portion of the cavity acting as the flow throttle.

## Revendications

1. Plaquette de circuits imprimés (100) avec au moins un canal de fluide (110), disposé à l'intérieur de la plaquette de circuits imprimés (100), comportant :
- une ouverture de canal de fluide (120) qui est disposée sur une surface de la plaquette de circuits imprimés (100) et mène vers le canal de fluide (110), et
- un système de soupape de sûreté (200) en plusieurs parties, qui est destiné à supprimer une surpression dans le canal de fluide (110) et qui comporte un premier aimant (210), un deuxième aimant (220) et une fermeture étanche (230),
- le premier aimant (210) et le deuxième aimant (220) étant disposés de telle sorte qu'ils pressent la fermeture étanche (230) sur l'ouverture de canal de fluide (120) sous l'effet d'une force magnétique (250) exercée par le premier aimant (210) et par le deuxième aimant (220), et
- le système de soupape de sûreté (200) est configuré de telle sorte qu'après une surpression dans le canal de fluide (110), il retourne automatiquement dans la position fermée.

2. Plaquette de circuits imprimés (100) selon la revendication 1, dans laquelle le premier aimant (210) est fixé sur la fermeture étanche (230) et le deuxième aimant (220) est fixé sur une face de la plaquette de circuits imprimés (100), opposée à l'ouverture de canal de fluide (120).

3. Plaquette de circuits imprimés (100) selon la revendication 1 ou 2, dans laquelle le premier aimant (210) et le deuxième aimant (220) sont disposés de telle sorte que la force magnétique (250) s'oppose à un déplacement du premier aimant (210) pour sortir d'une position centrale.

4. Plaquette de circuits imprimés (100) selon l'une quelconque des revendications précédentes, dans laquelle le premier aimant (210) et le deuxième aimant (220) comportent des surfaces (212, 222) orientées l'une vers l'autre, lesquelles sont disposées en étant sensiblement centrées et sensiblement perpendiculaires à un axe vertical (260) imaginaire et ont sensiblement la même forme.

5. Plaquette de circuits imprimés (100) selon l'une quelconque des revendications précédentes, dans laquelle le deuxième aimant (220) est incorporé dans un évidement de la plaquette de circuits imprimés (100).

6. Plaquette de circuits imprimés (100) selon l'une quelconque des revendications précédentes, dans laquelle le premier aimant (210) et le deuxième aimant (220) ont chacun un corps plat, dans lequel la largeur est sensiblement supérieure à la hauteur.

7. Plaquette de circuits imprimés (100) selon l'une quelconque des revendications précédentes, dans laquelle la fermeture étanche (230) comporte un élément d'étanchéité (240) qui, dans la position fermée du système de soupape de sûreté (200), est incorporé dans des évidements de la plaquette de circuits imprimés (100).

8. Plaquette de circuits imprimés (100) selon l'une quelconque des revendications précédentes, dans laquelle la fermeture étanche (230) comporte un élément d'étanchéité (240) en forme de joint torique.

9. Plaquette de circuits imprimés (100) selon l'une quelconque des revendications précédentes, comportant un composant électronique (270), tel qu'un composant électronique passif, tel qu'une résistance ou un condensateur, ou tel qu'un composant électronique actif, tel qu'un transistor ou un circuit de commande intégré, qui est disposé de telle sorte qu'il shunte le premier aimant (210) et est adapté pour empêcher que, en présence d'une surpression, la distance entre le premier aimant (210) et le deuxième aimant (220) soit supérieure à une distance maximale.

10. Plaquette de circuits imprimés (100) selon l'une quelconque des revendications précédentes, dans laquelle le premier aimant (210) et le deuxième aimant (220) sont disposés de telle sorte que, dans la position fermée du système de soupape de sûreté (200), ils ne sont pas en contact direct avec un fluide dans le canal de fluide (110).

11. Plaquette de circuits imprimés (100) selon l'une quelconque des revendications précédentes, ladite plaquette de circuits imprimés (100) étant une plaquette multicouche.

12. Insufflateur (500) comportant une plaquette de circuits imprimés (100) selon l'une des revendications précédentes.

13. Insufflateur (500) selon la revendication 12, comprenant un raccord d'admission (122) et un raccord d'évacuation (514) et un dispositif de mesure de la pression et de l'écoulement, disposé entre ces deux raccords et destiné à déterminer une pression de gaz appliquée sur le raccord d'évacuation (514) et à déterminer des grandeurs de mesure caractérisant un flux volumique de gaz appliqué sur le raccord d'évacuation (514), et qui comporte la plaquette de circuits imprimés (100), dans lequel :
- le canal de fluide (110) de la plaquette de circuits imprimés (100) est relié au niveau de son entrée au raccord d'admission (122) et au niveau de sa sortie au raccord d'évacuation (514), et
- des capteurs de mesure de pression (532, 534) et des composants électroniques (504, 506) pour la connexion des capteurs de mesure de pression (532, 534) sont disposés sur la plaquette de circuits imprimés (100), les capteurs de mesure de pression (532, 534) étant chacun en liaison directe avec le canal de fluide (110) via une ouverture correspondante dans une couche de la plaquette de circuits imprimés et étant configurés pour fournir une valeur de sortie représentant la pression statique régnant sur le lieu de l'ouverture respective.

14. Insufflateur (500) selon la revendication 13, dans lequel le canal de fluide (110) est formé par une cavité dans la plaquette de circuits imprimés (100), qui est formée de telle sorte qu'elle comporte une portion, qui agit sous forme de clapet d'étranglement et permet une mesure du flux volumique selon le principe du pneumotachographe.

15. Insufflateur (500) selon la revendication 14, dans lequel, au niveau de l'entrée et de la sortie de la portion de la cavité, agissant comme clapet d'étranglement, est disposé respectivement un capteur de pression (532, 534), lesquels sont reliés à des composants électroniques (504, 506) et sont adaptés à déterminer une différence entre la pression statique à l'entrée de la portion de la cavité, agissant comme clapet d'étranglement, et la pression statique à la sortie de la portion de la cavité, agissant comme clapet d'étranglement.
